# EUROPÄISCHE PATENTANMELDUNG

(11) **EP 3 763 338 A1**
(43) Veröffentlichungstag der Anmeldung: **13.01.2021**
(21) Anmeldenummer: 19185654.1
(22) Anmeldetag: 11.07.2019
(51) Int. Cl.: A61F 2/82

(54) **VERFAHREN ZUM MONTIEREN EINES RÖNTGENMARKERS IN EINEN STENT**

(71) Anmelder: BIOTRONIK AG, 8180 Bülach (CH)
(72) Erfinder: Risch, Fabian, 8200 Schaffhausen (CH)
(74) Vertreter: Galander, Marcus

(57) **Zusammenfassung**

Die Erfindung betrifft ein Verfahren zum Verbinden eines Röntgenmarkers (1) mit einem Grundkörper (20) eines Stents (2), aufweisend die Schritte: Bereitstellen eines Grundkörpers (20) eines Stents (2), wobei der Grundkörper (20) eine Durchgangsöffnung (21) zur Aufnahme eines Röntgenmarkers (1) aufweist; Bereitstellen einer flexiblen Folie (3) und eines Röntgenmarkers (1), so dass sich zumindest ein Abschnitt (30) der Folie (3) zwischen dem Grundkörper (20) mit der Durchgangsöffnung (21) und dem Röntgenmarker (1) befindet; und Einpressen des Röntgenmarkers (1) in die Durchgangsöffnung (21) des Grundkörpers (20) unter Zwischenlage der Folie (3), so dass der Röntgenmarker (1) plastisch verformt wird und zusammen mit der Folie (3) kraftschlüssig in der Durchgangsöffnung (21) festgelegt wird, wobei die Folie (3) einen Kontakt zwischen dem Röntgenmarker (1) und dem Grundkörper (20) verhindert.

## Beschreibung

Die Erfindung betrifft ein Verfahren zum Verbinden eines Röntgenmarkers mit einem Grundkörper eines Implantats, insbesondere eines Stents. Die vorliegende Erfindung wird hauptsächlich am Beispiel eines Röntgenmarkers für einen Stent beschrieben, ist jedoch nicht darauf eingeschränkt. Die Erfindung ist zum Verbinden eines Markers mit einem beliebigen, insbesondere biodegradierbaren, Implantats, wie beispielsweise Stents, Okkluder, Filter, Herzklappenprothesen (insbesondere stentbasierte Aortenklappenprothesen) oder sonstige Gefäßstützen.

Bei derartigen Verfahren wird bislang der Röntgenmarker z.B. mittels einer Klebung oder einer Pressung mit dem Stent verbunden.

Bei einer Klebung ergeben sich dabei die Probleme, dass sich zum einen die exakte Dosierung einer vergleichsweise sehr geringen Menge Klebstoff schwierig gestaltet, wobei zum anderen eine exakte Positionierung der Klebstoffmenge ebenfalls eine Herausforderung darstellt. Weiterhin liegt bei einer Klebung grundsätzlich das Problem einer eingeschränkten Verarbeitungsdauer (Topfzeit) vor. Weiterhin muss bei einer Verwendung von lösungsmittelbasierten Klebstoffen sichergestellt werden, dass sich diese nicht nachteilig auf das Implantat bzw. den Stent auswirken.

Weiterhin besteht bei einer Pressung grundsätzlich die Problematik, dass Röntgenmarker oftmals aus edlen Metallen gefertigt verwendet (z.B. Gold oder Platin...), die in Kombination mit einem unedlen Grundkörper des Stents eine Kontaktkorrosion erzeugen. Dies kann insbesondere bei biodegradierbaren Stents zu einer ungewünschten Beschleunigung der Degradation führen. Zusätzlich kann sich der Röntgenmarker aus dem Stent herauslösen, so dass ein direkter Kontakt zwischen dem Röntgenmarker und dem Grundkörper verhindert werden muss. Das gilt für alle Fügemethoden mit direktem Kontakt (z.B. Verschrauben, Nieten, etc.).

Die US 2016/0228267 offenbart das Einpressen eines Röntgenmarkers in einen Stent, wobei ein Zwischenraum zwischen dem Röntgenmarker und dem Stent mit einem Polymer gefüllt werden kann wenn der Stent beschichtet wird.

Der Erfindung liegt hiervon ausgehend die Aufgabe zugrunde, einen Röntgenmarker auf einfache und prozesssichere Weise mit einem Implantat, insbesondere einem Stent, zu verbinden, wobei eine mögliche Kontaktkorrosion von vorneherein vermieden werden soll. Insbesondere soll hierbei auf eine Klebeverbindung verzichtet werden.

Diese Aufgabe wird durch ein Verfahren mit den Merkmalen des Anspruchs 1 gelöst. Vorteilhafte Ausgestaltungen des Verfahrens sind in den entsprechenden Unteransprüchen angegeben und werden nachfolgend beschrieben.

Gemäß Anspruch 1 wird ein Verfahren zum Verbinden eines Röntgenmarkers mit einem Grundkörper eines Implantats, insbesondere eines Stents, offenbart, aufweisend die Schritte:
- Bereitstellen eines Grundkörpers eines Implantats, das eine Aufnahme, insbesondere eine Durchgangsöffnung, zur Aufnahme eines Röntgenmarkers aufweist,
- Bereitstellen einer flexiblen Folie und eines Röntgenmarkers, so dass sich zumindest ein Abschnitt der Folie zwischen dem Grundkörper mit der Aufnahme, insbesondere der Durchgangsöffnung ,und dem Röntgenmarker befindet,
- Einpressen des Röntgenmarkers in die Aufnahme, insbesondere die Durchgangsöffnung, des Grundkörpers unter Zwischenlage der Folie, so dass der Röntgenmarker plastisch verformt wird und zusammen mit der Folie kraftschlüssig in der Aufnahme, insbesondere der Durchgangsöffnung, festgelegt wird, wobei die Folie einen Kontakt zwischen dem Röntgenmarker und dem Grundkörper verhindert.

Die solchermaßen realisierte Verbindung zwischen dem Röntgenmarker und dem Grundkörper des Implantats ist dabei hinreichend elastisch, um eine leichte Deformation des Grundkörpers des Implantats ausgleichen zu können. Weiterhin kann das Einpressen insbesondere mit Vorteil so gestaltet werden, dass die Wandstärke des Grundkörpers durch den eingepressten Röntgenmarker nicht vergrößert wird.

Bevorzugt ist die Aufnahme als Hohlraum oder besonders bevorzugt als Durchgangsöffnung ausgestaltet.

Der Grundkörper kann eine Vielzahl an miteinander verbundenen Streben aufweisen, die eine umlaufende Gitterstruktur bilden. Ein derartiger Grundkörper kann bereitgestellt werden, indem einzelne Bereiche aus einem rohrförmigen Rohling z.B. mittels eines Lasers herausgeschnitten werden. Gemäß einer besonders bevorzugten Ausführungsform besteht der Grundkörper aus einer Magnesiumlegierung, insbesondere einer Mg-Zn-Ca, Mg-Zn-Al oder Mg-Al Legierung.

Gemäß einer Ausführungsform des Verfahrens ist vorgesehen, dass der Röntgenmarker so unter Zwischenlage der Folie in den Hohlraum oder die Durchgangsöffnung eingepresst wird, dass der Röntgenmarker mit einer umlaufenden Außenseite die Folie kontaktiert, die ihrerseits an einer umlaufenden Innenseite des Hohlraums oder der Durchgangsöffnung anliegt, so dass ein umlaufender Abschnitt der Folie zwischen dem Röntgenmarker und dem Grundkörper angeordnet ist.

Unter Hohlraum wird im Rahmen dieser Anmeldung jede mindestens einseitige Öffnung im Grundkörper des Implantats verstanden.

Weiterhin ist gemäß einer Ausführungsform des Verfahrens vorgesehen, dass der Röntgenmarker nach dem Einpressen in den Hohlraum/die Durchgangsöffnung eine entlang der Öffnungsebene des Hohlraums/der Durchgangsöffnung erstreckte Oberfläche aufweist, die mit einem Abschnitt der Folie überdeckt ist, der mit dem in die Durchgangsöffnung eingepressten umlaufenden Abschnitt der Folie verbunden ist. Der Röntgenmarker ist somit mit anderen Worten nach dem Einpressen in die Folie eingebettet, wobei lediglich eine weitere Oberfläche des Röntgenmarkers, die der folienbedeckten Oberfläche abgewandt ist, freiliegt.

Weiterhin ist gemäß einer Ausführungsform des Verfahrens vorgesehen, dass ein aus dem Hohlraum/der Durchgangsöffnung herausstehender Abschnitt der Folie abgetrennt wird, so dass vorzugsweise der umlaufende Abschnitt der Folie auf einer Seite des Grundkörpers flächenbündig mit einer Oberfläche des Grundkörpers abschließt bzw. angeordnet ist.

Gemäß einer weiteren Ausführungsform des Verfahrens ist vorgesehen, dass der herausstehende Abschnitt der Folie durch eine Stanzwirkung während des Einpressens entfernt bzw. abgetrennt wird. Gemäß einer alternativen Ausführungsform des Verfahrens ist vorgesehen, dass der herausstehende Abschnitt der Folie nachträglich zum Einpressen, zum Beispiel durch Schneiden, entfernt bzw. abgetrennt wird.

Gemäß einer alternativen Ausführungsform des erfindungsgemäßen Verfahrens ist vorgesehen, dass die Folie so bemessen ist, dass nach dem Einpressen des Röntgenmarkers in den Grundkörper die Folie auf einer Seite des Grundkörpers nicht aus dem Hohlraum/der Durchgangsöffnung des Grundkörpers herausragt.

Weiterhin ist gemäß einer Ausführungsform des Verfahrens vorgesehen, dass der Röntgenmarker vor dem Einpressen in die Durchgangsöffnung kugelförmig oder zylinderförmig ausgebildet ist. Gemäß einer Ausführungsform des Verfahrens ist weiterhin vorgesehen, dass der Röntgenmarker nach dem Einpressen in den Hohlraum / die Durchgangsöffnung aufgrund seiner plastischen Verformung halbschalenförmig / scheibenförmig ausgebildet ist und vorzugsweise mit seinen beiden einander abgewandten Oberflächen jeweils flächenbündig mit einer benachbarten Oberfläche des Grundkörpers ausgebildet ist.

Zum Einpressen des Röntgenmarkers können zwei Pressbacken verwendet werden, wobei der Röntgenmarker zusammen mit der Folie zwischen den Pressbacken angeordnet wird und die Pressbacken zum Einpressen des Röntgenmarkers und der Folie in den Hohlraum / die Durchgangsöffnung aufeinander zu bewegt werden, so dass der mindestens eine Röntgenmarker durch die beidseitig angreifenden Pressbacken plastisch verformt und dabei in dem Hohlraum / der Durchgangsöffnung kraftschlüssig festgelegt wird. Hierbei kann gemäß einer Ausführungsform die Bewegung der Pressbacken aufeinander zu so gesteuert werden, dass eine finale Dicke des mindestens einen Röntgenmarkers in der Pressrichtung einer Wandstärke des Grundkörpers entspricht (siehe oben).

Die verwendete Folie ist vorzugsweise derart elastisch oder plastisch verformbar ausgebildet, dass sie die beim Einpressen des Röntgenmarkers entstehenden Kräfte aufnehmen kann, ohne zu reißen.

Gemäß einer Ausführungsform des Verfahrens ist vorgesehen, dass die Folie eines der folgenden Materialien aufweist oder aus einem der folgenden Materialien gebildet ist: ein Kunststoff, ein Polymer, Polyurethan, elektrogesponnener Kunststoff, elektrogesponnenes Polyurethan, PTFE, Silikon.

Weiterhin ist gemäß einer Ausführungsform der Erfindung vorgesehen, dass der Röntgenmarker aus einem metallischen Material gebildet ist, das edler ist als ein metallisches Material, aus dem der Grundkörper gebildet ist. Der Röntgenmarker kann gemäß einer Ausführungsform aus einem der folgenden Materialien gebildet sein oder kann eines der folgenden Materialien aufweisen: ein röntgenopakes metallisches Material, Gold, eine Goldlegierung, Platin, eine Platinlegierung.

Gemäß einer weiteren Ausführungsform des Verfahrens ist vorgesehen, dass die Folie nach dem Einpressen des mindestens einen Röntgenmarkers aufgeschmolzen wird, z.B. um eine Schneidkante der Folie zu verrunden, oder um das Material der Folie gleichmäßig in einem Ringspalt um den mindestens einen Röntgenmarker herum zu verteilen.

Weiterhin ist gemäß einer Ausführungsform der Erfindung vorgesehen, dass die Folie beim Einpressen des mindestens einen Röntgenmarkers und der Folie in den Hohlraum / die Durchgangsöffnung aufgeschmolzen wird. Hierzu können z.B. die Pressbacken aufgeheizt werden, mit denen der mindestens eine Röntgenmarker und die Folie in die Durchgangsöffnung eingepresst werden.

Gemäß einer weiteren Ausführungsform des Verfahrens ist vorgesehen, dass der Röntgenmarker bzw. mehrere Röntgenmarker und die Folie vorkonfektioniert werden.

Gemäß einer Ausführungsform des Verfahrens ist diesbezüglich vorgesehen, dass das Bereitstellen der flexiblen Folie und des Röntgenmarkers vor dem Einpressen vorgenommen wird, indem der Röntgenmarker nebst weiteren Röntgenmarkern auf einer Folie angeordnet und dort geeignet festgelegt werden. Sodann kann das Einpressen der einzelnen Röntgenmarkern erfolgen.

Gemäß einer Ausführungsform ist diesbezüglich vorgesehen, dass das Bereitstellen der flexiblen Folie und des Röntgenmarkers vor dem Einpressen vorgenommen wird, indem die Folie auf einer Lochplatte angeordnet wird, die eine Mehrzahl an Löchern aufweist, wobei der Röntgenmarker und weitere Röntgenmarker jeweils auf einem Folienabschnitt, der ein Loch überdeckt, angeordnet und dort festgelegt werden, wobei der jeweilige Folienabschnitt aufgrund des darunter angeordneten Lochs der Lochplatte eine Auswölbung erhält, wobei vor dem Einpressen des Röntgenmarkers in den Hohlraum / die Durchgangsöffnung des Grundkörpers eine Auswölbung eines Folienabschnitts mit dem darauf festgelegten Röntgenmarker in der mindestens einen Durchgangsöffnung angeordnet wird.

Gemäß einer weiteren alternativen Ausführungsform des Verfahrens ist vorgesehen, dass das Bereitstellen der flexiblen Folie und des Röntgenmarkers vor dem Einpressen vorgenommen wird, indem der Röntgenmarker und weitere Röntgenmarker nebeneinander angeordnet werden und mit einem Kunststoff, vorzugsweise Polyurethan, zur Ausbildung der Folie elektrobesponnen werden.

Unter Elektrospinnen versteht man dabei die Erzeugung dünner Kunststoff- bzw. Polymerfasern aus einer Kunststoff- bzw. Polymerlösung in einem elektrischen Feld. Hierzu kann die Lösung zwischen einer Elektrode und einer Gegenelektrode beschleunigt werden, wobei die Lösung in einem komplexen Prozess in kleinste Fasern aufgespalten wird, die sich schließlich auf der Gegenelektrode ablagern, die Röntgenmarker können hierbei als Gegenelektrode fungieren so dass sie direkt mit dem Folienmaterial besponnen werden können.

Gemäß einer weiteren alternativen Ausführungsform des Verfahrens ist vorgesehen, dass das Bereitstellen der flexiblen Folie und des Röntgenmarkers vor dem Einpressen vorgenommen wird, indem der Röntgenmarker und weitere Röntgenmarker zwischen zwei die Folie bildenden Folienlagen der Folie angeordnet und an der Folie festgelegt werden.

Ein weiterer Aspekt der vorliegenden Erfindung betrifft ein Implantat, insbesondere einen Stent, aufweisend einen Grundkörper und einen in einer Aufnahme, insbesondere einer Durchgangsöffnung, des Grundkörpers angeordneten Röntgenmarker, der mittels des erfindungsgemäßen Verfahrens mit dem Grundkörper verbunden ist.

Im Folgenden sollen Ausführungsformen der Erfindung sowie weitere Merkmale und Vorteile anhand der Figuren erläutert werden. Es zeigen:
- Fig. 1A: eine schematische Schnittansicht eines Abschnitts eines Grundkörpers eines Stents mit einer Durchgangsöffnung, wobei oberhalb der Durchgangsöffnung ein kugelförmiger Röntgenmarker angeordnet ist, und wobei zwischen dem Marker und dem Grundkörper eine Folie angeordnet ist, die zusammen mit dem Marker in die Durchgangsöffnung eingepresst werden soll;
- Fig. 1B: eine schematische Schnittansicht des Röntgenmarkers gemäß Fig. 1A, der zusammen mit einem Abschnitt der Folie in der Durchgangsöffnung des Grundkörpers angeordnet wird;
- Fig. 2: eine schematische Schnittansicht des Röntgenmarkers, der Folie sowie des Grundkörpers, wobei zwei Pressbacken aufeinander zu verfahren werden, um den Röntgenmarker zusammen mit der Folie in die Durchgangsöffnung einzupressen;
- Fig. 3-4: die plastische Deformation des Röntgenmarkers gemäß Fig. 2 durch die Pressbacken;
- Fig. 5: den in die Durchgangsöffnung des Grundkörpers eingepressten Röntgenmarker, wobei ein Abschnitt der Folie nach dem Einpressen aus der Durchgangsöffnung heraussteht;
- Fig. 6: eine schematische Schnittansicht des Röntgenmarkers, der Folie sowie des Grundkörpers nach dem Einpressen des Röntgenmarkers in die Durchgangsöffnung des Grundkörpers, wobei der aus der Durchgangsöffnung herausstehende Abschnitt der Folie entfernt worden ist;
- Fig. 7A: REM Aufnahmen eines eingepressten Röntgenmarkers aus Gold in eine Folie aus elektrogesponnenem Polyurethan;
- Fig. 7B: REM Aufnahmen eines eingepressten Röntgenmarkers in aufgeschmolzenem Polyurethan in einer Ansicht von außerhalb des Grundkörpers;
- Fig. 7C: REM Aufnahmen eines eingepressten Röntgenmarkers in aufgeschmolzenem Polyurethan in einer Ansicht von innerhalb des Grundkörpers;
- Fig. 8A-8C: schematische Schnittansichten des Vormontierens von Röntgenmarkern auf einer Folie über einer Lochplatte; und
- Fig. 9A-9B: das schematische Einfügen der Röntgenmarker nach den Fig. 8A-8C in die Durchgangsöffnung eines Grundkörpers.

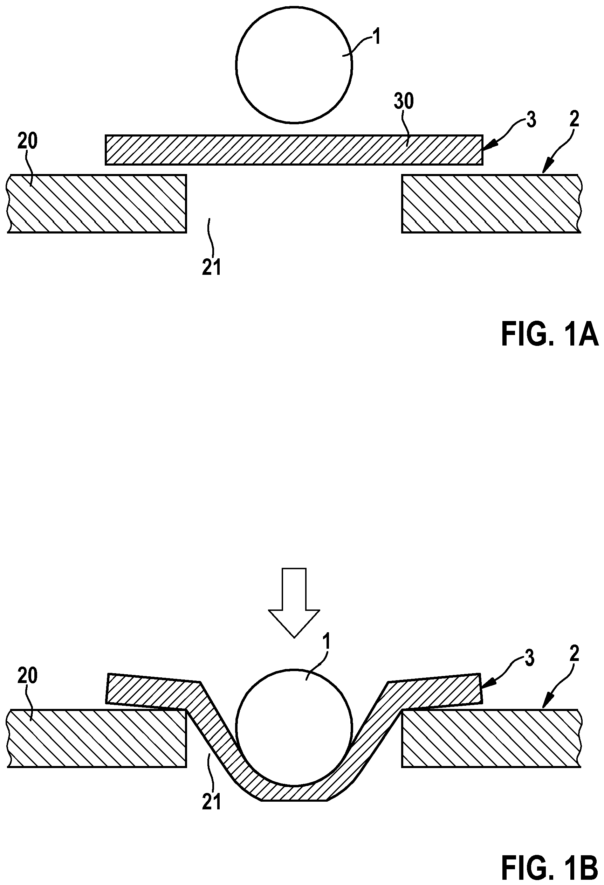

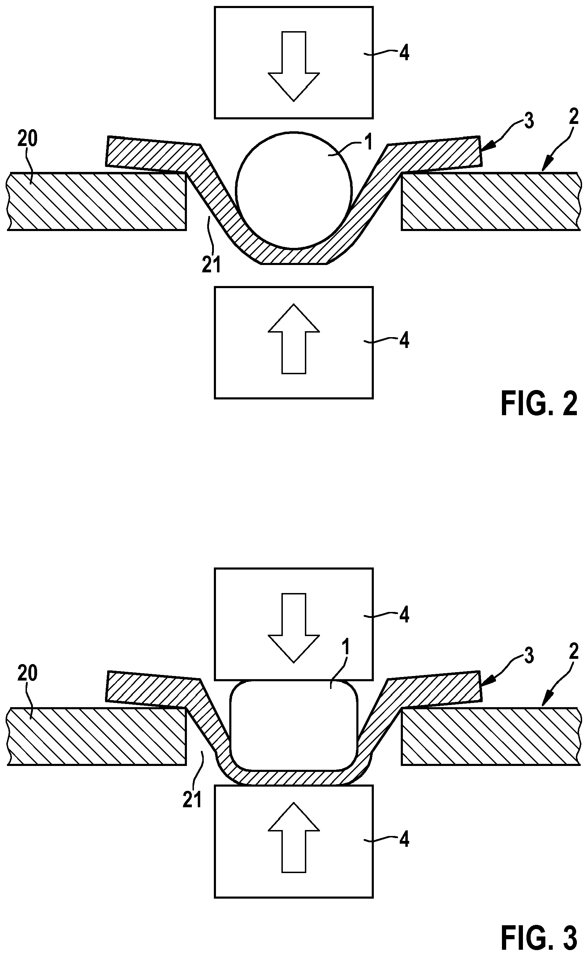

Die Figuren 1A und 1B zeigen im Zusammenhang mit den Figuren 2 bis 6 eine Ausführungsform eines erfindungsgemäßen Verfahrens zum Verbinden eines Röntgenmarkers 1 mit einem Grundkörper 20 eines Stents 2, wobei ein Grundkörpers 20 eines Stents 2 bereitgestellt wird, wobei der Grundkörper 20 eine Durchgangsöffnung 21 zur Aufnahme des Röntgenmarkers 1 aufweist, und wobei eine flexible Folie und ein Röntgenmarker 1 bereitgestellt werden, so dass sich zumindest ein Abschnitt 30 der Folie 3 zwischen dem Grundkörper 20 mit der Durchgangsöffnung 21 und dem Röntgenmarker 1 befindet, und wobei schließlich der Röntgenmarker 1 in die Durchgangsöffnung 21 des Grundkörpers (20) unter Zwischenlage der Folie 3 eingepresst wird, so dass der Röntgenmarker 1 plastisch verformt wird und zusammen mit der Folie 3 kraftschlüssig in der Durchgangsöffnung 21 festgelegt wird, wobei die Folie 3 einen Kontakt zwischen dem Röntgenmarker 1 und dem Grundkörper 20 verhindert und damit eine Kontaktkorrosion ausschließt.

Die Erfindung beschreibt somit eine alternative Montagemöglichkeit von Röntgenmarkern 1 in Stents 2. Herkömmlicherweise werden diese mittels Klebstoffdosierung eingeklebt. Die vorliegende Erfindung erlaubt indes das Herstellen einer Kunststoff- bzw. Polymerverbindung zwischen dem Marker 1 und dem Grundkörper 20 ohne jegliche Klebstoffdosierung.

Die verwendete Folie 3 ist vorzugsweise entsprechend elastisch verformbar und dazu ausgebildet, die beim Einpressen des Markers 1 entstehenden Kräfte aufnehmen können, ohne zu reißen. Ein Folienmaterial mit diesen Eigenschaften ist z.B. elektrogesponnenes Polyurethan. Grundsätzlich kommen alle elektrospinnbaren Kunststoffe in Frage, oder aber auch ein- oder mehrlagige PTFE- oder Silikon-Folien.

Eine solche Folie 3 wird gemäß Fig. 1A über einen Grundkörper 20 des Stents 2. Der Grundkörper 20 und insbesondere das oder die vorgeformten Durchgangsöffnungen 21, die auch als Markerlöcher oder "Eyelets" bezeichnet werden, sind dabei so ausgerichtet, dass ein Marker 1 von oben blind positioniert werden kann, das heißt, ohne dass die zugeordnete Durchgangsöffnung 21 durch die Folie 3 sichtbar sein muss. Eine Folie aus elektrogesponnenem Polyurethan ist in der Regel nur wenig transparent. Alternativ kann z.B. eine Lichtquelle unterhalb der Durchgangsöffnung 21 platziert werden. Durch den entsprechenden Schattenwurf wird dann die Position der jeweiligen Durchgangsöffnung 21 sichtbar. Weiterhin kann auch ein Gegenstößel durch die betreffende Durchgangsöffnung geschoben werden, der dann den Marker 1 während des Pressvorgangs führt. Fig. 1B zeigt den in der Durchgangsöffnung 21 zusammen mit der Folie 3 angeordneten Röntgenmarker 1 vor dem Einpressvorgang, der in den Figuren 2 bis 4 dargestellt ist.

Danach wird der Röntgenmarker 1, der zunächst vorzugsweise eine Kugelform aufweist und z.B. aus Gold bestehen kann (oder einem anderen stark röntgenopaken und weichem Metall, wie z.B. Platin) mit der Folie 3 mittels Pressbacken 4 in die Durchgangsöffnung 21 eingepresst. Über eine weggesteuerte Bewegung der Klemmbacken wird kontrolliert, dass die finale Dicke D der Markerscheibe 1 der Wandstärke W des Grundkörpers 20 des Stents 2 entspricht. Dadurch kann sichergestellt werden, dass der Grundkörper durch die Pressbewegung nicht verletzt wird, und auch dass der oder die Röntgenmarker 1 nicht über die Grundkörperkante hinausstehen.

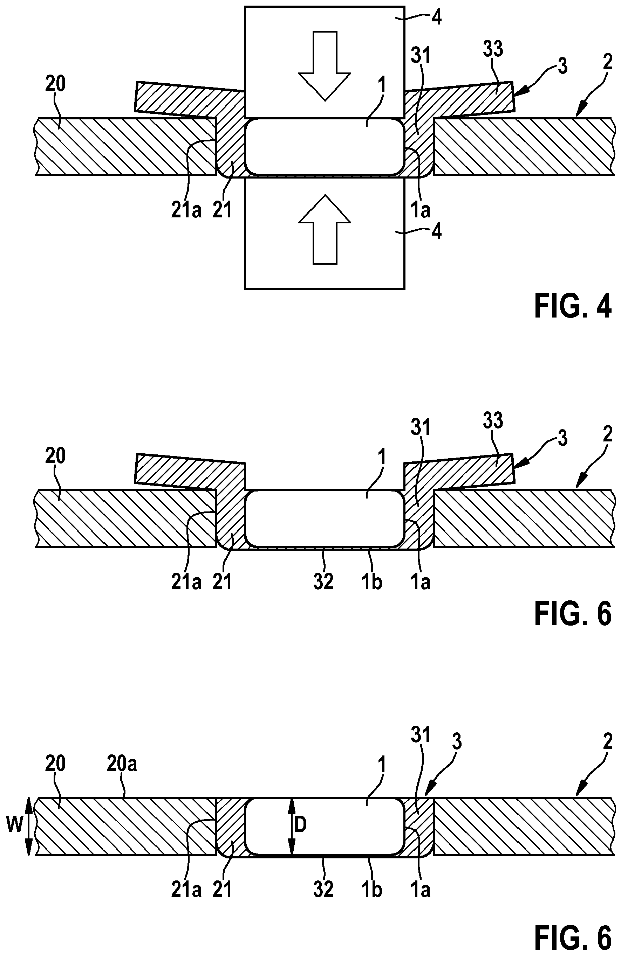

Die Folie 3 kann eine entsprechende Größe aufweisen, so dass nach dem Pressvorgang kein überstehender Folienabschnitt 33 vorhanden ist. Oder aber der überstehende Rest 33 der Folie 3 wird entfernt. Dies kann durch eine Stanzwirkung während des Pressens realisiert werden oder nach dem Einpressen, zum Beispiel durch Abschneiden des überstehenden Abschnitts 33 der Folie 3 (vgl. Figuren 5 und 6).

Gemäß Fig. 6 ist der Röntgenmarker im Ergebnis vorzugsweise so unter Zwischenlage der Folie 3 in die Durchgangsöffnung 21 eingepresst, dass der Röntgenmarker 1 mit einer umlaufenden Außenseite 1a die Folie 3 kontaktiert, die ihrerseits an einer umlaufenden Innenseite 21a der Durchgangsöffnung 21 anliegt, so dass ein umlaufender Abschnitt 31 der Folie zwischen dem Röntgenmarker 1 und dem Grundkörper 20 angeordnet ist bzw. einen entsprechenden Ringspalt zwischen dem Marker 1 und dem Grundkörper 20 ausfüllt.

Gemäß Fig. 6 ist weiterhin vorgesehen, dass nach dem Einpressen in die Durchgangsöffnung (21) der Röntgenmarker 1 eine entlang der Öffnungsebene der Durchgangsöffnung 21 erstreckte Oberfläche 1b aufweist, die mit einem Abschnitt 32 der Folie 3 überdeckt ist, der mit dem in die Durchgangsöffnung 21 eingepressten umlaufenden Abschnitt 31 der Folie 3 verbunden ist.

Zusätzlich kann die eingepresste Folie 3 nachträglich noch aufgeschmolzen werden. Z.B., um eine Schneidkante der Folie 3 zu verrunden, oder um das Folienmaterial (z.B. Polyurethan) gleichmäßig in dem Ringspalt zu verteilen. Das Aufschmelzen kann in die Pressbewegung integriert werden, zum Beispiel durch aufheizbare Pressbacken 4.

Als Pressbacken kommen z.B. Flachzangen in Frage, deren Pressbewegung mechanisch limitiert ist, so dass nicht weiter als die Wandstärke des Stents gepresst werden kann.
Die Pressbewegung kann auch in den bestehenden Prozessschritt des Crimpens integriert werden. Unter Crimpen wird in Rahmen dieser Anmeldung der Prozessschritt verstanden, bei dem der Stent um ein Expansionsmittel (beispielsweise ein dilatierbarer Ballon eines Ballonkatheters) angeordnet ist und der Durchmesser des Stents auf de Durchmesser für die Einführung in den Körper reduziert wird. Der Stent wird auf den Ballon komprimiert.

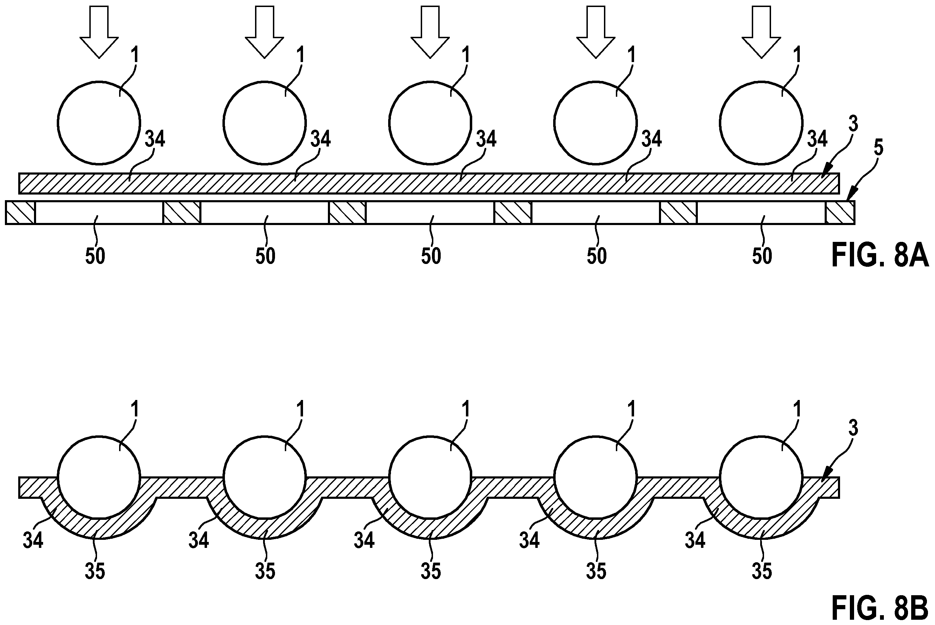

In einer alternativen Ausführungsform, die z.B. exemplarisch in den Figuren 8A bis 9 dargestellt ist, wird vor dem eigentlichen Montageschritt bzw. dem Einpressen eines Markers 1 in eine zugeordnete Durchgangsöffnung 21 des Grundkörpers 20 eine Baugruppe erzeugt, bei der Marker 1 auf der Folie 3 vorpositioniert und dort insbesondere festgelegt sind.

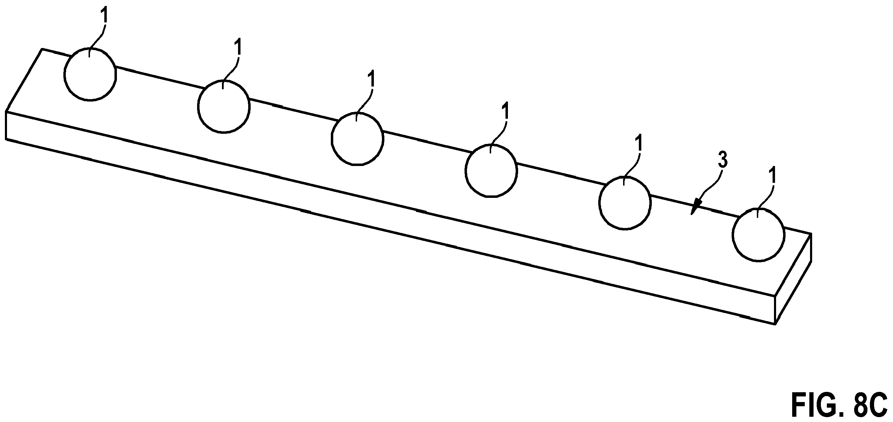

Dabei werden zum Beispiel auf einer Folie 3, z.B. in Form eines Folienstreifens 3, mehrere Röntgenmarker 1 über einer Lochplatte (z.B. in Form eines Lochblechs) vormontiert (siehe Fig. 8A), so dass jeweils ein Röntgenmarker 1 auf einem Folienabschnitt 34 angeordnet wird, der ein Loch 50 der Lochplatte 5 verdeckt und in dieses hereingedrückt wird, so dass sich jeweils eine Auswölbung 35 der Folie 3 ergibt, die einen Marker 1 aufnimmt (vgl. Figuren 8B und 8C).

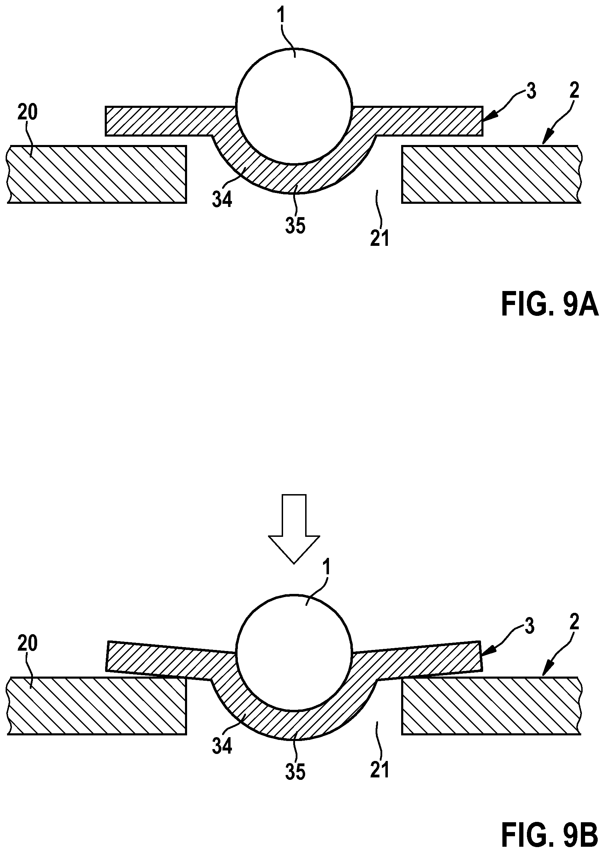

Diese Auswölbungen 35 können dann besonders leicht gemäß Figuren 9A und 9B zusammen mit dem jeweils darin angeordneten Röntgenmarker 1 in der zugeordneten Durchgangsöffnung 21 des Grundkörpers 20 positioniert werden. Hiernach kann dann der Einpressvorgang durchgeführt werden, z.B. gemäß den Figuren 2 bis 6 (siehe oben).

Weiterhin kann eine solche Baugruppe aus Marken 1 und Folie 3 erzeugt werden, indem mehrere Röntgenmarker 1 direkt mit einem geeigneten Kunststoff/Polymer (z.B. Polyurethan) elektrobesponnen werden. Alternativ hierzu besteht die Möglichkeit mehrere Röntgenmarker 1 beidseitig mit einer Folienlage (z.B. Polyurethan) zu bedecken, so dass die Marker 1 in eine Folie 3 aus zumindest zwei Folienlagen eingepackt sind.

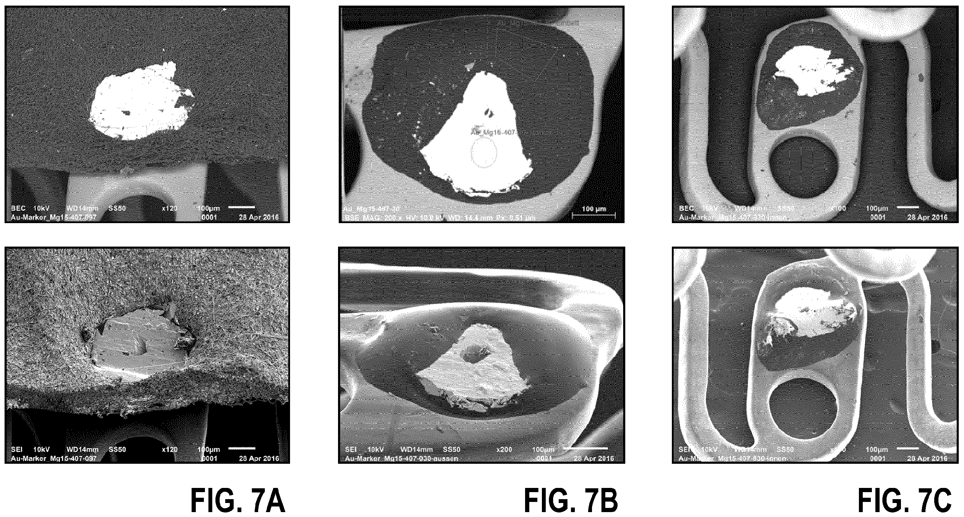

Die Figuren 7A bis 7C zeigen REM Aufnahmen von Röntgenmarkern, die mittels der vorliegenden Erfindung in die gezeigten Stents eingepresst worden sind.

Die Vorteile der vorliegenden Erfindung liegen darin, dass auf eine Klebstoffdosierung (mit den einhergehenden Nachteilen, siehe oben) vollständig verzichtet werden kann. Es können dabei kostengünstige Röntgenmarker im Ursprungszustand (kugelförmig) verwendet werden, da sie keine engen Toleranzen aufweisen müssen. Ferner kann der Montageschritt in den Crimpprozess integriert werden.

## Patentansprüche

1. Verfahren zum Verbinden eines Röntgenmarkers (1) mit einem Grundkörper (20) eines Implantats, insbesondere eines Stents (2), aufweisend die Schritte:
- Bereitstellen eines Grundkörpers (20) eines Stents (2), wobei der Grundkörper (20) eine Aufnahme, insbesondere eine Durchgangsöffnung (21), zur Aufnahme eines Röntgenmarkers (1) aufweist,
- Bereitstellen einer flexiblen Folie (3) und eines Röntgenmarkers (1), so dass sich zumindest ein Abschnitt (30) der Folie (3) zwischen dem Grundkörper (20) mit der Aufnahme, insbesondere der Durchgangsöffnung (21), und dem Röntgenmarker (1) befindet, und
- Einpressen des Röntgenmarkers (1) in die Aufnahme, insbesondere in die Durchgangsöffnung (21), des Grundkörpers (20) unter Zwischenlage der Folie (3), so dass der Röntgenmarker (1) plastisch verformt wird und zusammen mit der Folie (3) kraftschlüssig in der Aufnahme, insbesondere der Durchgangsöffnung (21), festgelegt wird, wobei die Folie (3) einen Kontakt zwischen dem Röntgenmarker (1) und dem Grundkörper (20) verhindert.

2. Verfahren nach Anspruch 1, **wobei** die Aufnahme als ein Hohlraum oder eine Durchgangsöffnung (21) ausgestaltet ist.

3. Verfahren nach Anspruch 2, **wobei** der Röntgenmarker (1) so unter Zwischenlage der Folie (3) in den Hohlraum oder die Durchgangsöffnung (21) eingepresst wird, dass der Röntgenmarker (1) mit einer umlaufenden Außenseite (1a) die Folie (3) kontaktiert, die ihrerseits an einer umlaufenden Innenseite (21a) des Hohlraums oder der Durchgangsöffnung (21) anliegt, so dass ein umlaufender Abschnitt (31) der Folie zwischen dem Röntgenmarker (1) und dem Grundkörper (20) angeordnet ist.

4. Verfahren nach Anspruch 3, **wobei** der Röntgenmarker (1) nach dem Einpressen in den Hohlraum oder die Durchgangsöffnung (21) eine entlang der Öffnungsebene des Hohlraums oder der Durchgangsöffnung (21) erstreckte Oberfläche (1b) aufweist, die mit einem Abschnitt (32) der Folie (3) überdeckt ist, der mit dem in den Hohlraum oder die Durchgangsöffnung (21) eingepressten umlaufenden Abschnitt (31) der Folie (3) verbunden ist.

5. Verfahren nach einem der vorhergehenden Ansprüche, **wobei** ein aus dem Hohlraum oder der Durchgangsöffnung (21) herausstehender Abschnitt (33) der Folie (3) entfernt wird, so dass vorzugsweise der umlaufende Abschnitt (31) der Folie (3) auf einer Seite des Grundkörpers (20) flächenbündig mit einer Oberfläche (20a) des Grundkörpers (20) abschließt.

6. Verfahren nach einem der Ansprüche 1 bis 4, **wobei** die Folie (3) so bemessen ist, dass nach dem Einpressen die Folie (3) auf einer Seite des Grundkörpers (20) nicht aus dem Hohlraum oder der Durchgangsöffnung (21) des Grundkörpers (20) herausragt.

7. Verfahren nach einem der vorhergehenden Ansprüche, **wobei** der Röntgenmarker (1) vor dem Einpressen in die Durchgangsöffnung (21) kugel- oder zylinderförmig ausgebildet ist, und/oder wobei der Röntgenmarker (1) nach dem Einpressen in den Hohlraum oder die Durchgangsöffnung (21) aufgrund seiner plastischen Verformung halbschalenförmig oder scheibenförmig ausgebildet ist.

8. Verfahren nach einem der vorhergehenden Ansprüche, **wobei** der Röntgenmarker (1) mittels zweier Pressbacken (4) zusammen mit der Folie (3) in den Hohlraum oder die Durchgangsöffnung (21) eingepresst wird, wobei der Röntgenmarker (1) zusammen mit der Folie (3) zwischen den Pressbacken (4) angeordnet wird und die Pressbacken (4) zum Einpressen des Röntgenmarkers (1) und der Folie (3) in den Hohlraum oder die Durchgangsöffnung (21) aufeinander zu bewegt werden, so dass der mindestens eine Röntgenmarker (1) durch die beidseitig angreifenden Pressbacken (4) plastisch verformt und dabei in dem Hohlraum oder der Durchgangsöffnung (21) kraftschlüssig festgelegt wird.

9. Verfahren nach Anspruch 8, **wobei** die Bewegung der Pressbacken (4) aufeinander zu so gesteuert wird, dass eine finale Dicke (D) des mindestens einen Röntgenmarkers (1) einer Wandstärke (W) des Grundkörpers (20) entspricht.

10. Verfahren nach einem der vorhergehenden Ansprüche, **wobei** die Folie (3) eines der folgenden Materialien aufweist oder aus einem der folgenden Materialien gebildet ist: ein Kunststoff, ein Polymer, Polyurethan, elektrogesponnener Kunststoff, elektrogesponnenes Polyurethan, PTFE, Silikon.

11. Verfahren nach einem der vorhergehenden Ansprüche, **wobei** der Röntgenmarker (1) aus einem metallischen Material gebildet ist, das edler ist als ein metallisches Material, aus dem der Grundkörper (20) gebildet ist.

12. Verfahren nach einem der vorhergehenden Ansprüche, **wobei** der Röntgenmarker (1) aus einem der folgenden Materialien gebildet ist oder eines der folgenden Materialien aufweist: ein röntgenopakes metallisches Material, Gold, eine Goldlegierung, Platin, eine Platinlegierung.

13. Verfahren nach einem der vorhergehenden Ansprüche, **wobei** dass die Folie (3) nach dem Einpressen des mindestens einen Röntgenmarkers (1) aufgeschmolzen wird.

14. Verfahren nach Anspruch 13, **wobei** die Folie (3) beim Einpressen des mindestens einen Röntgenmarkers (1) und der Folie (3) in den Hohlraum oder die Durchgangsöffnung (21) aufgeschmolzen wird.

15. Verfahren nach einem der vorhergehenden Ansprüche, **wobei** das Bereitstellen der flexiblen Folie (3) und des Röntgenmarkers (1) vorgenommen wird, indem
- die Folie (3) auf einer Lochplatte (5) angeordnet wird, die eine Mehrzahl an Löchern (50) aufweist, wobei der Röntgenmarker (1) und weitere Röntgenmarker (1) jeweils auf einem Folienabschnitt (34), der ein Loch (50) überdeckt, angeordnet und dort festgelegt werden, wobei der jeweilige Folienabschnitt (34) aufgrund des darunter angeordneten Lochs (50) der Lochplatte (5) eine Auswölbung (35) erhält, und wobei vor dem Einpressens des Röntgenmarkers (1) in den Hohlraum oder die Durchgangsöffnung (21) des Grundkörpers (20) eine Auswölbung (35) eines Folienabschnitts (34) mit dem darauf festgelegten Röntgenmarker (1) in dem Hohlraum oder der Durchgangsöffnung (21) angeordnet wird, oder indem
- der Röntgenmarker (1) und weitere Röntgenmarker (1) nebeneinander angeordnet werden und mit einem Kunststoff, vorzugsweise Polyurethan, zur Ausbildung der Folie elektrobesponnen werden, oder indem
- der Röntgenmarker (1) und weitere Röntgenmarker (1) zwischen zwei die Folie (3) bildenden Folienlagen der Folie (3) angeordnet werden.

16. Implantat, insbesondere Stent (2), aufweisend einen Grundkörper (20) und einen in einer Aufnahme, insbesondere Durchgangsöffnung (21), des Grundkörpers (20) angeordneten Röntgenmarker (1), der mittels des Verfahrens nach einem der vorhergehenden Ansprüche mit dem Grundkörper (20) verbunden ist.
